# NEUE EUROPÄISCHE PATENTSCHRIFT

(11) **EP 3 218 474 B2**
(45) Veröffentlichungstag und Bekanntmachung des Hinweises auf die Entscheidung über den Einspruch: **22.05.2024**
(45) Hinweis auf die Patenterteilung: 15.01.2020
(21) Anmeldenummer: 15787565.9
(22) Anmeldetag: 28.10.2015
(51) Int. Cl.: C12N 9/54, C11D 3/386

(54) **WASCH- UND REINIGUNGSMITTEL, ENTHALTEND MINDESTENS ZWEI PROTEASEN**
DETERGENTS AND CLEANERS, COMPRISING AT LEAST TWO PROTEASES
DÉTERGENTS ET AGENTS DE LAVAGES CONTENANT AU MOINS DEUX PROTÉASES

(30) Priorität: 14.11.2014 DE 102014223296
(43) Veröffentlichungstag der Anmeldung: 20.09.2017
(73) Patentinhaber: Henkel AG & Co. KGaA, 40589 Düsseldorf (DE)
(72) Erfinder: O'CONNELL, Timothy, 40547 Düsseldorf (DE); TONDERA, Susanne, 40597 Düsseldorf (DE); WEBER, Thomas, 41541 Dormagen (DE)
(86) Internationale Anmeldenummer: PCT/EP2015/074971
(87) Internationale Veröffentlichungsnummer: WO 2016/074925

(56) Entgegenhaltungen:
- WO-A1-91/02792
- WO-A1-95/23221
- WO-A1-2013/076269
- WO-A1-2013/076269
- WO-A1-2014/177430
- WO-A1-2014/177430
- WO-A2-2009/021867
- WO-A2-2009/021867
- US-A- 4 511 490
- US-A- 5 352 604
- US-A- 5 677 272
- US-A1- 2012 238 005
- US-B2- 7 510 859
- Henkel letter to EPO dated 18.02.2002
- Copy of the IPRP
- Henkel letter to EPO dated 28.11.2017, with EN translation
- Experimental evidence
- Smith et al. 1968
- Uniprot entry for P29600
- Sequence alignments
- Alignment between D4 fig 27 amino acid sequence and SEQ ID NO 1.

## Beschreibung

Die Erfindung betrifft Mittelzusammensetzungen, insbesondere Wasch- und Reinigungsmittel, die eine erste Protease und mindestens eine zweite Protease enthalten, gegebenenfalls auch noch eine dritte oder weitere Proteasen. Weiterhin betrifft die Erfindung Reinigungsverfahren, in denen diese Mittel eingesetzt sind, Verwendungen dieser Mittel sowie Reinigungsverfahren und Verwendungen dieser Proteasen.

Proteasen gehören zu den technisch bedeutendsten Enzymen überhaupt. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren den Serin-Proteasen zugerechnet werden. Sie wirken als unspezifische Endopeptidasen, das heißt, sie hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegegeben von R. Bott und C. Betzel, New York, 1996. Subtilasen werden natürlicherweise von Mikroorganismen gebildet; hierunter sind insbesondere die von Bacillus-Spezies gebildeten und sekretierten Subtilisine als bedeutendste Gruppe innerhalb der Subtilasen zu erwähnen.

Proteasen sind neben anderen Enzymen etablierte aktive Inhaltsstoffe von Wasch- und Reinigungsmitteln. Sie bewirken dabei den Abbau proteinhaltiger Anschmutzungen auf dem Reinigungsgut. Günstigenfalls ergeben sich Synergieeffekte zwischen den Enzymen und den übrigen Bestandteilen der betreffenden Mittel. Unter den Wasch- und Reinigungsmittelproteasen nehmen Subtilasen aufgrund ihrer günstigen enzymatischen Eigenschaften wie Stabilität oder pH-Optimum eine herausragende Stellung ein. Sie eignen sich daneben noch für eine Vielzahl weiterer technischer Verwendungsmöglichkeiten, beispielsweise als Bestandteile von Kosmetika oder in der organisch-chemischen Synthese.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7.

Das Subtilisin BPN', welches aus Bacillus amyloliquefaciens, beziehungsweise B. subtilis stammt, ist aus den Arbeiten von Vasantha et al. (1984) in J. Bacteriol., Volume 159, S. 811-819 und von J. A. Wells et al. (1983) in Nucleic Acids Research, Volume 11, S. 7911-7925 bekannt. Subtilisin BPN' dient insbesondere hinsichtlich der Numerierung der Positionen als Referenzenzym der Subtilisine. Subtilisin Carlsberg ist in weiterentwickelter Form unter dem Handelsnamen Alcalase^{®} von der Firma Novozymes A/S, Bagsvaerd, Dänemark, erhältlich. Sie wird in den Publikationen von E. L. Smith et al. (1968) in J. Biol. Chem., Volume 243, S. 2184-2191, und von Jacobs et al. (1985) in Nucl. Acids Res., Band 13, S. 8913-8926 beschrieben und wird natürlicherweise von Bacillus licheniformis gebildet. Die Protease PB92 wird natürlicherweise von dem alkaliphilen Bakterium Bacillus nov. spec. 92 produziert und war unter dem Handelsnamen Maxacal^{®} von der Fa. Gist-Brocades, Delft, Niederlande, erhältlich. In ihrer ursprüglichen Sequenz wird sie in der Patentanmeldung EP 283075 A2 beschrieben. Die Subtilisine 147 und 309 werden unter den Handelsnamen Esperase^{®}, beziehungsweise Savinase^{®} von der Firma Novozymes vertrieben. Sie stammen ursprünglich aus Bacillus-Stämmen, die mit der Anmeldung GB 1243784 A offenbart werden. Von der Protease aus Bacillus lentus DSM 5483 (WO 91/02792 A1) leiten sich die unter der Bezeichnung BLAP^{®} geführten Varianten ab, die insbesondere in WO 92/21760 A1, WO 95/23221 A1, WO 02/088340 A2 und WO 03/038082 A2 beschrieben werden. Subtilisin DY ist ursprünglich von Nedkov et al. 1985 in Biol. Chem Hoppe-Seyler, Band 366, S. 421-430 beschrieben. Weitere verwendbare Proteasen sind beispielsweise die unter den Handelsnamen Durazym^{®}, Relase^{®}, Everlase^{®}, Nafizym, Natalase^{®}, Kannase^{®} und Ovozyme^{®} von der Firma Novozymes, die unter den Handelsnamen, Purafect^{®}, Purafect^{®} OxP, Purafect^{®} Prime und Properase^{®} von der Firma Genencor, das unter dem Handelsnamen Protosol^{®} von der Firma Advanced Biochemicals Ltd., Thane, Indien, das unter dem Handelsnamen Wuxi^{®} von der Firma Wuxi Snyder Bioproducts Ltd., China, die unter den Handelsnamen Proleather^{®} und Protease P^{®} von der Firma Amano Pharmaceuticals Ltd., Nagoya, Japan, und das unter der Bezeichnung Proteinase K-16 von der Firma Kao Corp., Tokyo, Japan, erhältlichen Enzyme. Weitere in Wasch-und Reinigungsmittel verwendbare Protease-Varianten sind z.B. in WO2013076269A1, WO2014177430A1 und WO2009021867A2 beschrieben.

Die in erfindungsgemäßen Mitteln eingesetzten Proteasen stammen entweder ursprünglich aus Mikroorganismen, beispielsweise aus Mikroorganismen der Gattungen Bacillus, Streptomyces, Humicola, oder Pseudomonas, und/oder werden nach an sich bekannten biotechnologischen Verfahren durch geeignete Mikroorganismen produziert, beispielsweise durch transgene Expressionswirte der Gattungen Bacillus oder durch filamentöse Fungi.

Komplexe Protein-Anschmutzungen bzw. -Rückstände stellen hohe Anforderungen an die in dem jeweiligen Mittel enthaltene Protease. Beispielsweise werden Anschmutzungen durch eine in dem Mittel enthaltene Protease nicht vollständig bzw. zufrieden stellend entfernt. Auch können weitere Inhaltsstoffe der Anschmutzung die Leistung einer Protease herabsetzen. Um die Leistungsfähigkeit von Mitteln, insbesondere Wasch- und Reinigungsmitteln, hinsichtlich ihrer proteolytischen Aktivität zu verbessern, wird beispielsweise versucht, neue Proteasen mit vorteilhaften Eigenschaften zu gewinnen, indem beispielsweise Mikroorganismen-haltige Proben aus natürlichen Habitaten entnommen werden und unter den als geeignet angesehenen Bedingungen - zum Beispiel in alkalischem Milieu - zu kultivieren. Auch ist es möglich, Proteasen über an sich bekannte Mutagenese-Verfahren für den Einsatz in den jeweiligen Mitteln, insbesondere Wasch-und Reinigungsmitteln, zu optimieren. Dazu gehören Punktmutagenese, Deletion, Insertion oder Fusion mit anderen Proteinen oder Proteinteilen oder über sonstige, insbesondere chemische Modifikationen. Beispielsweise werden hierdurch die Oberflächenladungen und/oder den isoelektrischen Punkt der Moleküle und darüber ihre Wechselwirkungen mit dem Substrat verändert oder die Stabilität der betreffenden Proteasen und damit ihre Wirksamkeit erhöht. Jedoch weisen die hier beispielhaft dargestellten Weiterentwicklungsmöglichkeiten der in entsprechenden Mitteln einsetzbaren Proteasen den Nachteil auf, dass sie komplex und zeitaufwändig sind.

Hinsichtlich der Anwendung von Proteasen in Wasch- und Reinigungsmitteln ist bekannt, dass Proteasen zur Verbesserung der Wasch- beziehungsweise Reinigungsleistung zusammen mit weiteren Enzymen, beispielsweise Amylasen, Cellulasen, Hemicellulasen, Mannanasen, β-Glucosidasen, Oxidasen, Oxidoreduktasen oder Lipasen eingesetzt werden können. Ebenso ist der Einsatz von Proteasen in Waschmitteln in Kombination mit anderen Wirkstoffen wie etwa Bleichmitteln oder Soil-Release-Wirkstoffen dem Fachmann bekannt. Aus dem Stand der Technik ist ferner bekannt, dass entsprechende Mittel, insbesondere Wasch- und Reinigungsmittel, mehrere Proteasen enthalten können. So offenbart die internationale Patentanmeldung WO 03/054185 A1, dass die Alkalische Protease aus Bacillus gibsonii (DSM 14391) in einem entsprechenden Mittel zusammen mit weiteren Proteasen eingesetzt werden kann. Allerdings enthält diese Anmeldung wie auch der weitere Stand der Technik keinen Hinweis darauf, mit welchen spezifischen Proteasen dieses Enzym vorteilhafterweise zu kombinieren ist, um ein synergistisches Zusammenwirken der Proteasen in einem entsprechenden Mittel zu erzielen.

Überraschenderweise wurde gefunden, dass die Leistungsfähigkeit von Mitteln, insbesondere Wasch- und Reinigungsmitteln, hinsichtlich ihrer proteolytischen Aktivität signifikant verbessert wird, wenn in diesen Mitteln Mischungen von mindestens zwei bestimmten Proteasen mit unterschiedlichem Wirkspektrum und/oder unterschiedlicher Sequenz eingesetzt sind. Hinsichtlich des Zusammenwirkens von diesen bestimmten, unterschiedlichen Proteasen ergibt sich eine synergistische Wirkung, d.h. eine bessere Leistung im Vergleich mit den Einzelleistungen der jeweiligen Protease in Ein-Komponenten-Systemen (d.h. Mitteln, die nur jeweils diese Proteasen enthalten) und auch gegenüber der Summe der Einzelleistungen der Proteasen, d.h. der Summe von beispielsweise zwei Ein-Komponenten-Systemen. Somit stellt die ausgewählte Kombination von bestimmten Proteasen eine weitere Möglichkeit dar, um die Leistungsfähigkeit von Mitteln, insbesondere Wasch- und Reinigungsmitteln, hinsichtlich ihrer proteolytischen Aktivität zu verbessern.

Der vorliegenden Erfindung liegt daher die Aufgabe zugrunde, Mittel bereitzustellen, die eine verbesserte proteolytische Aktivität aufweisen.

Hiermit verbunden ist eine weitere Aufgabe der vorliegenden Erfindung, Mittel bereitzustellen, die eine verbesserte Wasch- bzw. Reinigungsleistung, insbesondere bei niedrigen Temperaturen, bevorzugter maßen bei etwa 20°C, in Bezug auf zumindest eine proteinhaltige Anschmutzung, vorzugsweise in Bezug auf mehrere proteinhaltige Anschmutzungen, aufweisen.

Eine weitere besondere Aufgabe der vorliegenden Erfindung ist es, Mittel bereitzustellen, die eine mindestens gleich bleibende, vorzugsweise eine verbesserte Wasch- bzw. Reinigungsleistung in Bezug auf zumindest eine proteinhaltige Anschmutzung, vorzugsweise in Bezug auf mehrere proteinhaltige Anschmutzungen, aufweisen, aber eine reduzierten Gehalt an Protease aufweisen.

Diese Aufgabe wird erfindungsgemäß gelöst durch die Bereitstellung von Mitteln gemäß Anspruch 1, die Mischungen von speziell ausgewählten Proteasen mit unterschiedlichen Wirkspektren bzw. unterschiedlicher Sequenz enthalten. In diesen erfindungsgemäßen Mitteln entfalten die enthaltenen und dafür speziell ausgewählten Proteasen eine synergistische Reinigungsleistung. Daher bewirken die Mittel eine bessere Entfernung proteinhaltiger Anschmutzungen als Mittel, die entweder nur eine Protease enthalten, oder auch Mittel, die zwei Proteasen enthalten, aber deren Reinigungsleistung sich nur aus der bloßen Addition der jeweiligen Einzelbeiträge der beiden enthaltenen Proteasen ergibt. Somit stellt die ausgewählte Kombination von solchen Proteasen einen wesentlichen Aspekt der Erfindung dar, die in erfindungsgemäßen Mitteln eine synergistische Wirkung hinsichtlich der Entfernung von proteinhaltigen Rückständen oder Anschmutzungen aufweisen.

Einen Gegenstand der Erfindung stellen somit Mittel gemäß Anspruch 1 dar.

Das erfindungsgemäße Mittel kann zusätzlich zu der ersten und der zweiten Protease noch eine oder mehrere weitere Proteasen enthalten, die beliebige, von der ersten und der zweiten Protease verschiedene Proteasen sein können, vorzugsweise jedoch ausgewählt sind unter Proteasen, die eine Aminosäuresequenz umfassen, die zu einer der in SEQ ID NO.2 bis SEQ ID NO.7 angegebenen Aminosäuresequenzen mindestens zu 80% und zunehmend bevorzugt zu mindestens 82,5%, 85%, 87,5%, 90%, 91%, 92%, 93%, 94%, 95%, 96%, 97%, 98%, 99%, 99,5% und ganz besonders bevorzugt zu 100% identisch ist.

Erfindungsgemäße Mittel enthalten die oben beschriebenen mindestens zwei Proteasen insbesondere in Gesamtmengen von 0,05 bis 5 Gewichts-Prozent (Gew.-%), vorzugsweise 0,05 bis 2 Gew.-%, bezogen auf die Gesamtmenge des Mittels.

Bezogen auf aktives Protein enthalten die erfindungsgemäßen Mittel die oben beschriebenen mindestens zwei Proteasen insbesondere in Gesamtmengen von 0,05 bis 15 Gew.-%, vorzugsweise 0,05 bis 10 Gew.-%.

Alle Sachverhalte, Gegenstände und Ausführungsformen, die für erfindungsgemäße Wasch- oder Reinigungsmittel beschrieben sind, sind entsprechend auch auf die genannten Enzymgemische anwendbar. Daher wird an dieser Stelle ausdrücklich auf die Offenbarung an entsprechender Stelle verwiesen, mit dem Hinweis, dass diese Offenbarung auch für die vorstehenden erfindungsgemäßen Enzymgemische gilt.

Überraschenderweise bewirkt die erfindungsgemäße Kombination einer Protease, deren Aminosäuresequenz der in SEQ ID NO.1 angegebenen Aminosäuresequenz entspricht, mit mindestens einer anderen Protease, die ausgewählt ist einer Aminosäuresequenz entsprechen, die zu der in SEQ ID NO.6 oder SEQ ID NO.7 angegebenen Aminosäuresequenz mindestens zu 80% und zunehmend bevorzugt zu mindestens 82,5%, 85%, 87,5%, 90%, 91%, 92%, 93%, 94%, 95%, 96%, 97%, 98%, 99%, 99,5% und ganz besonders bevorzugt zu 100% identisch ist, in einem erfindungsgemäßen Mittel, eine verbesserte synergistische Reinigungsleistung. Die synergistische Wirkung beruht darauf, dass sich die Wirkungen der voneinander unterschiedlichen Proteasen so ergänzen, dass in der Gesamtheit eine gesteigerte proteolytische Wirkung erreicht wird.

Ebenfalls überraschenderweise wurde festgestellt, dass diese verbesserte Wirkung insbesondere bei niedrigen Temperaturen, vorzugsweise im Temperaturbereich von 10°C bis 30°C, besonders bevorzugt bei etwa 20°C eintritt.

Die verbesserte Wirkung kann beispielsweise dadurch ermöglicht werden, dass durch die proteolytische Spaltung von Peptidbindungen durch die erste Protease Substrate für die zweite Protease verbessert zugänglich werden und durch die proteolytische Spaltung der Peptidbindungen durch die zweite Protease wiederum weitere Substrate für die erste Protease zugänglich werden. Gerade bei komplexen proteinhaltigen Substraten ist die Zugänglichkeit einer Spaltstelle für eine Protease häufig ein limitierender Faktor. Ein unterschiedliches Wirkspektrum der beiden Proteasen zeigt sich daher beispielsweise in einer unterschiedlichen Substratspezifität. Ebenso kann ein unterschiedliches Wirkspektrum hervorgerufen sein durch eine unterschiedliche proteolytische Aktivität unter definierten Bedingungen, insbesondere hinsichtlich Temperatur, pH-Wert, Ionenstärke, Stabilität gegenüber oxidierenden Verbindungen (beispielsweise Bleichmitteln), usw.

Ein erfindungsgemäßer Synergismus tritt demnach dann auf, wenn sich die erste Protease von der zweiten Protease und den gegebenenfalls vorhandenen weiteren Proteasen unterscheidet. Diese Unterschiede manifestieren sich in der Aminosäuresequenz des jeweils proteolytisch aktiven Enzyms.

Unter einem Enzym ist im Sinne der vorliegenden Anmeldung ein Protein zu verstehen, das eine bestimmte biokatalytische Funktion ausübt. Unter Protease im Sinne der vorliegenden Anmeldung wird ein Enzym verstanden, welches die Hydrolyse von Peptidbindungen katalysiert und dadurch in der Lage ist, Peptide oder Proteine zu spalten.

Ein Protein ist im Sinne der vorliegenden Anmeldung ein aus den natürlichen Aminosäuren zusammengesetztes, weitgehend linear aufgebautes, zur Ausübung seiner Funktion zumeist eine dreidimensionale Struktur annehmendes Polypeptid. Ein Peptid besteht aus Aminosäuren, die über Peptidbindungen miteinander kovalent verbunden sind. Die Bezeichnung Polypeptid verdeutlicht diesbezüglich den Sachverhalt, dass diese Peptidkette in der Regel aus vielen Aminosäuren besteht, die über Peptidbindungen miteinander verbunden sind. Aminosäuren können in einer L-und einer D-Konfiguration vorliegen, wobei die Aminosäuren, aus denen Proteine bestehen, in der L-Konfiguration vorliegen. Sie werden als proteinogene Aminosäuren bezeichnet. In der vorliegenden Anmeldung werden die proteinogenen, natürlich vorkommenden L-Aminosäuren mit den international gebräuchlichen 1-und 3-Buchstaben-Codes bezeichnet. Zahlreiche Proteine werden als sogenannte Präproteine, also zusammen mit einem Signalpeptid gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signalpeptid unter natürlichen Bedingungen durch eine Signalpeptidase vom übrigen Protein abgespalten, so dass dieses seine eigentliche katalytische Aktivität ohne die zunächst vorhandenen N-terminalen Aminosäuren ausübt. Pro-Proteine sind inaktive Vorstufen von Proteinen. Deren Vorläufer mit Signalsequenz werden als Prä-Pro-Proteine bezeichnet. Für technische Anwendungen sind aufgrund ihrer enzymatischen Aktivität die maturen, d.h. reifen Peptide, d.h. die nach ihrer Herstellung prozessierten Enzyme gegenüber den Präproteinen bevorzugt.

Die Proteine können von den sie produzierenden Zellen nach der Herstellung der Polypeptidkette modifiziert werden, beispielsweise durch Anknüpfung von Zuckermolekülen, Formylierungen, Aminierungen, usw. Solche Modifikationen werden als posttranslationale Modifikationen bezeichnet. Diese posttranslationalen Modifizierungen können, müssen jedoch nicht einen Einfluss auf die Funktion des Proteins ausüben.

Durch Vergleich mit bekannten Enzymen, die beispielsweise in allgemein zugänglichen Datenbanken hinterlegt sind, lässt sich aus der Aminosäure- oder Nukleotid-Sequenz die enzymatische Aktivität eines betrachteten Enzyms folgern. Diese kann durch andere Bereiche des Proteins, die nicht an der eigentlichen Reaktion beteiligt sind, qualitativ oder quantitativ modifiziert werden. Dies könnte beispielsweise die Enzymstabilität, die Aktivität, die Reaktionsbedingungen oder die Substratspezifität betreffen.

Solch ein Vergleich geschieht dadurch, dass ähnliche Abfolgen in den Nukleotid- oder Aminosäuresequenzen der betrachteten Proteine einander zugeordnet werden. Dies nennt man Homologisierung. Eine tabellarische Zuordnung der betreffenden Positionen wird als Alignment bezeichnet. Bei der Analyse von Nukleotidsequenzen sind wiederum beide komplementären Stränge und jeweils allen drei möglichen Leserastern zu berücksichtigen; ebenso die Degeneriertheit des genetischen Codes und die organismenspezifische Verwendung der Codons (Codon-Usage). Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in Science, Band 227, S. 1435-1441 beschrieben. Alle in der vorliegenden Patentanmeldung durchgeführten Sequenzvergleiche und Bestimmungen von Homologie- und/oder Identitätswerten wurden mit dem Computer-Programm Vector NTI^{®} Suite 7.0, erhältlich von der Firma InforMax, Inc., Bethesda, USA, mit den vorgegebenen Default-Parametern durchgeführt.

Eine Zusammenstellung aller in den verglichenen Sequenzen übereinstimmenden Positionen wird als Konsensus-Sequenz bezeichnet. Solch ein Vergleich erlaubt auch eine Aussage über die Ähnlichkeit oder Homologie der verglichenen Sequenzen zueinander. Diese wird in Prozent Identität, das heißt dem Anteil der identischen Nukleotide oder Aminosäurereste an denselben bzw. in einem Alignment einander entsprechenden Positionen wiedergegeben. Ein weiter gefaßter Homologiebegriff bezieht die konservierten Aminosäure-Austausche in diesen Wert mit ein. Es ist dann von Prozent Ähnlichkeit die Rede. Solche Aussagen können über ganze Proteine oder Gene oder nur über einzelne Bereiche getroffen werden.

Homologe Bereiche von verschiedenen Proteinen sind durch Übereinstimmungen in der Aminosäuresequenz definiert. Diese können auch durch identische Funktion gekennzeichnet sein. Sie geht bis zu völligen Identitäten in kleinsten Bereichen, sogenannten Boxen, die nur wenige Aminosäuren umfassen und meist für die Gesamtaktivität essentielle Funktionen ausüben. Unter den Funktionen der homologen Bereiche sind kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes. Proteasen bzw. Enzyme im allgemeinen können durch verschiedene Verfahren, z.B. gezielte genetische Veränderung durch Mutageneseverfahren, weiterentwickelt und für bestimmte Einsatzzwecke oder hinsichtlich spezieller Eigenschaften, beispielsweise katalytische Aktivität, Stabilität, usw., optimiert werden.

Es ist aus dem Stand der Technik weiterhin allgemein bekannt, dass sich vorteilhafte Eigenschaften einzelner Mutationen, z.B. einzelner Punktmutationen, ergänzen können. Eine hinsichtlich bestimmter Eigenschaften bereits optimierte Protease, zum Beispiel hinsichtlich ihrer Stabilität gegenüber Tensiden oder anderen Komponenten, kann erfindungsgemäß zusätzlich weiterentwickelt werden.

Unter Fragmenten werden alle Proteine oder Peptide verstanden, die kleiner sind als natürliche Proteine und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Unter chimären oder hybriden Proteinen sind im Sinne der vorliegenden Anmeldung solche Proteine zu verstehen, deren Sequenz die Sequenzen oder Teilsequenzen von mindestens zwei Ausgangsproteinen umfasst. Die Ausgangsproteine können diesbezüglich aus verschiedenen oder aus demselben Organismus stammen. Chimäre oder hybride Proteine können beispielsweise durch Rekombinationsmutagenese erhalten werden. Der Sinn einer solchen Rekombination kann beispielsweise darin bestehen, mithilfe des heranfusionierten Proteinteils eine bestimmte enzymatische Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können.

Unter durch Insertionsmutation erhaltene Proteine sind solche Varianten zu verstehen, die durch Einfügen eines Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter Derivaten werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch die Wirtszelle erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise auch um andere Proteine handeln, die beispielsweise über bifunktionelle chemische Verbindungen an erfindungsgemäße Proteine gebunden werden. Derartige Modifizierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Ebenso ist unter Derivatisierung die kovalente Bindung an einen makromolekularen Träger zu verstehen, genauso wie auch ein nichtkovalenter Einschluss in geeignete makromolekulare Käfigstrukturen. In einer weiteren Ausführungsform der Erfindung ist das Mittel somit dadurch gekennzeichnet, dass die erste Protease in dem Mittel als Fragment, Deletionsvariante, chimäres Protein oder Derivat vorliegt und/oder die zweite Protease in dem Mittel als Fragment, Deletionsvariante, chimäres Protein oder Derivat vorliegt, wobei die erste und die zweite Protease weiterhin katalytisch aktiv sind.

Im Sinne der vorliegenden Erfindung werden alle Enzyme, Proteine, Fragmente, chimäre Proteine und Derivate, sofern sie nicht explizit als solche angesprochen zu werden brauchen, unter dem Oberbegriff Proteine zusammengefasst.

Erfindungsgemäße Mittel umfassen alle Arten von Mitteln, insbesondere Gemische, Rezepturen, Lösungen etc., deren Einsetzbarkeit durch Zugabe der oben beschriebenen Proteasen verbessert wird. Es kann sich dabei je nach Einsatzgebiet beispielsweise um feste Gemische, beispielsweise Pulver mit gefriergetrockeneten oder verkapselten Proteinen, oder um gelförmige oder flüssige Mittel handeln. Bevorzugte Rezepturen enthalten beispielsweise Puffersubstanzen, Stabilisatoren, Reaktionspartner und/oder Cofaktoren der Proteasen und/oder andere mit den Proteasen synergistische Inhaltsstoffe. Insbesondere sind darunter Mittel für die weiter unten ausgeführten Einsatzgebiete zu verstehen. Weitere Einsatzgebiete gehen aus dem Stand der Technik hervor und werden beispielsweise in dem Handbuch "Industrial enyzmes and their applications" von H. Uhlig, Wiley-Verlag, New York, 1998 dargestellt.

In bevorzugten Ausführungsformen der Erfindung ist ein erfindungsgemäßes Mittel dadurch gekennzeichnet, dass es ein Waschmittel, Handwaschmittel, Spülmittel, Handgeschirrspülmittel, Maschinengeschirrspülmittel, Reinigungsmittel, Zahnprothesen- oder Kontaktlinsenpflegemittel, Nachspülmittel, Desinfektionsmittel, kosmetisches Mittel, pharmazeutisches Mittel oder ein Mittel zur Behandlung von Filtermedien, Textilien, Pelzen, Papier, Fellen oder Leder, ist, insbesondere ein Wäschewaschmittel oder ein Geschirrspülmittel.

Zu diesem Erfindungsgegenstand zählen alle denkbaren Wasch- bzw. Reinigungsmittelarten, sowohl Konzentrate als auch unverdünnt anzuwendende Mittel, zum Einsatz im kommerziellen Maßstab, in der Waschmaschine oder bei der Hand-Wäsche beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispiels weise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet.

Ein erfindungsgemäßes Mittel kann sowohl ein Mittel für Großverbraucher oder technische Anwender als auch ein Produkt für den Privatverbraucher sein, wobei alle im Stand der Technik etablierten Wasch- und Reinigungsmittelarten ebenfalls Ausführungsformen der vorliegenden Erfindung darstellen.

Die erfindungsgemäßen Wasch- oder Reinigungsmittel, die als insbesondere pulverförmige Feststoffe, in nachverdichteter Teilchenform, als homogene Lösungen oder Suspensionen vorliegen können, können außer dem erfindungsgemäß eingesetzten Wirkstoff - einer erfindungsgemäßen Proteasekombination - im Prinzip alle bekannten und in derartigen Mitteln üblichen Inhaltsstoffe enthalten, wobei mindestens ein weiterer Inhaltsstoff in dem Mittel vorhanden ist. Die erfindungsgemäßen Mittel können insbesondere Buildersubstanzen, oberflächenaktive Tenside, Bleichmittel auf Basis organischer und/oder anorganischer Persauerstoffverbindungen, Bleichaktivatoren, wassermischbare organische Lösungsmittel, Enzyme, Sequestrierungsmittel, Elektrolyte, pH-Regulatoren und weitere Hilfsstoffe wie optische Aufheller, Vergrauungsinhibitoren, Schaumregulatoren sowie Farb- und Duftstoffe sowie Kombinationen hiervon enthalten. Insbesondere eine Kombination der Proteasekombination mit einem oder mehreren weiteren Inhaltsstoff(en) der Mittel erweist sich als vorteilhaft, da ein solches Mittel eine verbesserte Reinigungsleistung durch sich ergebende Synergismen, insbesondere zwischen einer Protease bzw. der Proteasekombination und dem weiteren Inhaltsstoff, aufweist. Dies bedeutet, dass das Mittel eine verbesserte Entfernung von Anschmutzungen, beispielsweise proteinhaltigen Anschmutzungen, bewirkt im Vergleich mit einem Mittel, welches entweder nur eine der beiden Komponenten beinhaltet, oder auch im Vergleich zur erwarteten Reinigungsleistung eines Mittels mit beiden Komponenten auf Grund der bloßen Addition der jeweiligen Einzelbeiträge dieser beiden Komponenten zur Reinigungsleistung des Mittels. Insbesondere durch die Kombination einer erfindungsgemäßen Proteasekombination mit einem der nachfolgend beschriebenen Tenside und/oder Buildersubstanzen und/oder Bleichmittel wird ein solcher Synergismus erreicht.

Die erfindungsgemäßen Mittel können ein Tensid oder mehrere Tenside enthalten, wobei insbesondere anionische Tenside, nichtionische Tenside und deren Gemische, aber auch kationische, zwitterionische und amphotere Tenside in Frage kommen.

Geeignete nichtionische Tenside sind insbesondere Alkylglykoside und Ethoxylierungs- und/oder Propoxylierungsprodukte von Alkylglykosiden oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkyl-aminen, vicinalen Diolen, Fettsäureestem und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett-oder Oleylalkohol, und durchschnittlich 2 bis 8 EO pro Mol Alkohol bevorzugt. Zu den bevorzugten ethoxylierten Alkoholen gehören beispielsweise C₁₂-C₁₄-Alkohole mit 3 EO oder 4 EO, C₉-C₁₁-Alkohole mit 7 EO, C₁₃-C₁₅-Alkohole mit 3 EO, 5 EO, 7 EO oder 8 EO, C₁₂-C₁₈-Alkohole mit 3 EO, 5 EO oder 7 EO und Mischungen aus diesen, wie Mischungen aus C₁₂-C₁₄-Alkohol mit 3 EO und C₁₂-C₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind (Talg-) Fettalkohole mit 14 EO, 16 EO, 20 EO, 25 EO, 30 EO oder 40 EO. Insbesondere in Mitteln für den Einsatz in maschinellen Verfahren werden üblicherweise extrem schaumarme Verbindungen eingesetzt. Hierzu zählen vorzugsweise C₁₂-C₁₈-Alkylpolyethylenglykol-polypropylenglykolether mit jeweils bei zu 8 Mol Ethylenoxid- und Propylenoxideinheiten im Molekül. Man kann aber auch andere bekannt schaumarme nichtionische Tenside verwenden, wie zum Beispiel C₁₂-C₁₈-Alkylpolyethylenglykol-polybutylenglykolether mit jeweils bis zu 8 Mol Ethylenoxid- und Butylenoxideinheiten im Molekül sowie endgruppenverschlossene Alkylpolyalkylenglykolmischether. Besonders bevorzugt sind auch die hydroxylgruppenhaltigen alkoxylierten Alkohole, wie sie in der europäischen Patentanmeldung EP 0 300 305 beschrieben sind, sogenannte Hydroxymischether. Zu den nichtionischen Tensiden zählen auch Alkylglykoside der allgemeinen Formel RO(G)ₓ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl - die als analytisch zu bestimmende Größe auch gebrochene Werte annehmen kann - zwischen 1 und 10; vorzugsweise liegt x bei 1,2 bis 1,4. Ebenfalls geeignet sind Polyhydroxyfettsäureamide der Formel (III), in der R¹CO für einen aliphatischen Acylrest mit 6 bis 22 Kohlenstoffatomen, R² für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht:

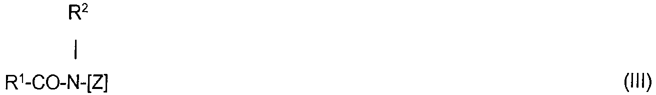

Vorzugsweise leiten sich die Polyhydroxyfettsäureamide von reduzierenden Zuckern mit 5 oder 6 Kohlenstoffatomen, insbesondere von der Glucose ab. Zur Gruppe der Polyhydroxyfettsäureamide gehören auch Verbindungen der Formel (IV), 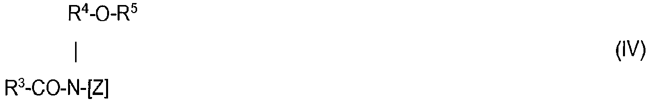 in der R³ für einen linearen oder verzweigten Alkyl- oder Alkenylrest mit 7 bis 12 Kohlenstoffatomen, R⁴ für einen linearen, verzweigten oder cyclischen Alkylenrest oder einen Arylenrest mit 2 bis 8 Kohlenstoffatomen und R⁵ für einen linearen, verzweigten oder cyclischen Alkylrest oder einen Arylrest oder einen Oxy-Alkylrest mit 1 bis 8 Kohlenstoffatomen steht, wobei C₁-C₄-Alkyl-oder Phenylreste bevorzugt sind, und [Z] für einen linearen Polyhydroxyalkylrest, dessen Alkylkette mit mindestens zwei Hydroxylgruppen substituiert ist, oder alkoxylierte, vorzugsweise ethoxylierte oder propoxylierte Derivate dieses Restes steht. [Z] wird auch hier vorzugsweise durch reduktive Aminierung eines Zuckers wie Glucose, Fructose, Maltose, Lactose, Galactose, Mannose oder Xylose erhalten. Die N-Alkoxy- oder N-Aryloxy-substituierten Verbindungen können dann beispielsweise durch Umsetzung mit Fettsäuremethylestern in Gegenwart eines Alkoxids als Katalysator in die gewünschten Polyhydroxyfettsäureamide überführt werden. Eine weitere Klasse bevorzugt eingesetzter nichtionischer Tenside, die entweder als alleiniges nichtionisches Tensid oder in Kombination mit anderen nichtionischen Tensiden, insbesondere zusammen mit alkoxylierten Fettalkoholen und/oder Alkylglykosiden, eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester. Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon. Als weitere Tenside kommen sogenannte Gemini-Tenside in Betracht. Hierunter werden im Allgemeinen solche Verbindungen verstanden, die zwei hydrophile Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, dass die hydrophilen Gruppen einen ausreichenden Abstand haben, damit sie unabhängig voneinander agieren können. Derartige Tenside zeichnen sich im Allgemeinen durch eine ungewöhnlich geringe kritische Micell-konzentration und die Fähigkeit, die Oberflächenspannung des Wassers stark zu reduzieren, aus. In Ausnahmefällen werden unter dem Ausdruck Gemini-Tenside nicht nur derartig "dimere", sondern auch entsprechend "trimere" Tenside verstanden. Geeignete Gemini-Tenside sind beispielsweise sulfatierte Hydroxymischether oder Dimeralkohol-bis- und Trimeralkohol-tris-sulfate und -ethersulfate. Endgruppenverschlossene dimere und trimere Mischether zeichnen sich insbesondere durch ihre Bi- und Multifunktionalität aus. So besitzen die genannten endgruppenverschlossenen Tenside gute Netzeigenschaften und sind dabei schaumarm, so dass sie sich insbesondere für den Einsatz in maschinellen Wasch- oder Reinigungsverfahren eignen. Eingesetzt werden können aber auch Gemini-Polyhydroxyfettsäureamide oder Poly-Polyhydroxyfettsäureamide. Geeignet sind auch die Schwefelsäuremonoester der mit 1 bis 6 Mol Ethylenoxid ethoxylierten geradkettigen oder verzweigten C₇-C₂₁-Alkohole, wie 2-Methylverzweigte C₉-C₁₁-Alkohole mit im Durchschnitt 3,5 Mol Ethylenoxid (EO) oder C₁₂-C₁₈-Fettalkohole mit 1 bis 4 EO. Zu den bevorzugten Aniontensiden gehören auch die Salze der Alkylsulfobernsteinsäure, die auch als Sulfosuccinate oder als Sulfobernsteinsäureester bezeichnet werden, und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈- bis C₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen. Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen. Als weitere anionische Tenside kommen Fettsäure-Derivate von Aminosäuren, beispielsweise von N-Methyltaurin (Tauride) und/oder von N-Methylglycin (Sarkoside) in Betracht. Insbesondere bevorzugt sind dabei die Sarkoside beziehungsweise die Sarkosinate und hier vor allem Sarkosinate von höheren und gegebenenfalls einfach oder mehrfach ungesättigten Fettsäuren wie Oleylsarkosinat. Als weitere anionische Tenside kommen insbesondere Seifen in Betracht. Geeignet sind insbesondere gesättigte Fettsäureseifen, wie die Salze der Laurinsäure, Myristinsäure, Palmitinsäure, Stearinsäure, hydrierten Erucasäure und Behensäure sowie insbesondere aus natürlichen Fettsäuren, zum Beispiel Kokos-, Palmkern- oder Talgfettsäuren, abgeleitete Seifengemische. Zusammen mit diesen Seifen oder als Ersatzmittel für Seifen können auch die bekannten Alkenylbernsteinsäuresalze eingesetzt werden.

Die anionischen Tenside, einschließlich der Seifen, können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Tenside sind in erfindungsgemäßen Mitteln in Mengenanteilen von vorzugsweise 5 Gew.-% bis 50 Gew.-%, insbesondere von 8 Gew.-% bis 30 Gew.-%, enthalten.

Ein erfindungsgemäßes Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, insbesondere die durch Oxidation von Polysacchariden beziehungsweise Dextrinen zugänglichen Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättiger Carbonsäuren liegt im allgemeinen zwischen 3 000 und 200 000, die der Copolymeren zwischen 2 000 und 200 000, vorzugsweise 30 000 bis 120 000, jeweils bezogen auf freie Säure. Ein besonders bevorzugtes Acrylsäure-Maleinsäure-Copolymer weist eine relative Molekülmasse von 30 000 bis 100 000 auf. Handelsübliche Produkte sind zum Beispiel Sokalan^{®} CP 5, CP 10 und PA 30 der Firma BASF. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/oder einem veresterten Vinylalkohol oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure, wobei Maleinsäure besonders bevorzugt ist, und/oder ein Derivat einer Allylsulfonsäure, die in 2-Stellung mit einem Alkyl- oder Arylrest substituiert ist, sein. Derartige Polymere weisen im Allgemeinen eine relative Molekülmasse zwischen 1 000 und 200 000 auf. Weitere bevorzugte Copolymere sind solche, die als Monomere vorzugsweise Acrolein und Acrylsäure/Acrylsäuresalze beziehungsweise Vinylacetat aufweisen. Die organischen Buildersubstanzen können, insbesondere zur Herstellung flüssiger Mittel, in Form wäßriger Lösungen, vorzugsweise in Form 30- bis 50-gewichtsprozentiger wäßriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt.

Als wasserlösliche anorganische Buildermaterialien kommen insbesondere Alkalisilikate, Alkalicarbonate und Alkaliphosphate, die in Form ihrer alkalischen, neutralen oder sauren Natriumoder Kaliumsalze vorliegen können, in Betracht. Beispiele hierfür sind Trinatriumphosphat, Tetranatriumdiphosphat, Dinatriumdihydrogendiphosphat, Pentanatriumtriphosphat, sogenanntes Natriumhexametaphosphat, oligomeres Trinatriumphosphat mit Oligomerisierungsgraden von 5 bis 1000, insbesondere 5 bis 50, sowie die entsprechenden Kaliumsalze beziehungsweise Gemische aus Natrium- und Kaliumsalzen. Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Natriumalumosilikate in Waschmittelqualität, insbesondere Zeolith A, P und gegebenenfalls X, allein oder in Mischungen, beispielsweise in Form eines Co-Kristallisats aus den Zeolithen A und X (Vegobond^{®} AX, ein Handelsprodukt der Condea Augusta S.p.A.), bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das nach den Angaben der deutschen Patentschrift DE 24 12 837 bestimmt werden kann, liegt in der Regel im Bereich von 100 bis 200 mg CaO pro Gramm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁·y H₂O eingesetzt, in der x, das sogenannte Modul, eine Zahl von 1,9 bis 22, insbesondere 1,9 bis 4 und y eine Zahl von 0 bis 33 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisitikate (Na₂Si₂O₅·yH₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Kristalline schichtförmige Silikate der oben angegebenen Formel (I) werden von der Fa. Clariant GmbH unter dem Handelsnamen Na-SKS vertrieben, z.B. Na-SKS-1 (Na₂Si₂₂O₄₅·xH₂O, Kenyait), Na-SKS-2 (Na₂Si₁₄O₂₉xH₂O, Magadiit), Na-SKS-3 (Na₂SisO₁₇·xH₂O) oder Na-SKS-4 (Na₂Si₄O₉·xH₂O, Makatit). Von diesen eignen sich vor allem Na-SKS-5 (α-Na₂Si₂O₅), Na-SKS-7 (β-Na₂Si₂O₅, Natrosilit), Na-SKS-9 (NaHSi₂O₅·3H₂O), Na-SKS-10 (NaHSi₂O₅·3H₂O, Kanemit), Na-SKS-11 (t-Na₂Si₂O₅) und Na-SKS-13 (NaHSi₂O₅), insbesondere aber Na-SKS-6 (δ-NazSi₂O₅). In einer bevorzugten Ausgestaltung erfindungsgemäßer Mittel setzt man ein granulares Compound aus kristallinem Schichtsilikat und Citrat, aus kristallinem Schichtsilikat und oben genannter (co-)polymerer Polycarbonsäure, oder aus Alkalisilikat und Alkalicarbonat ein, wie es beispielsweise unter dem Namen Nabion^{®} 15 im Handel erhältlich ist. Buildersubstanzen sind in den erfindungsgemäßen Mitteln vorzugsweise in Mengen bis zu 75 Gew.-%, insbesondere 5 Gew.-% bis 50 enthalten.

Als für den Einsatz in erfindungsgemäßen Mitteln geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Waschbedingungen Wasserstoffperoxid abgebende anorganische Salze, zu denen Perborat, Percarbonat, Persilikat und/oder Persulfat wie Caroat gehören, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Falls ein erfindungsgemäßes Mittel Persauerstoffverbindungen enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren beschriebene Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Die hydrophil substituierten Acylacetale und die Acyllactame werden ebenfalls bevorzugt eingesetzt. Auch Kombinationen konventioneller Bleichaktivatoren können eingesetzt werden. Derartige Bleichaktivatoren können, insbesondere bei Anwesenheit obengenannter Wasserstoffperoxid-liefernder Bleichmittel, im üblichen Mengenbereich, vorzugsweise in Mengen von 0,5 Gew.-% bis 10 Gew.-%, insbesondere 1 Gew.-% bis 8 Gew.-%, bezogen auf gesamtes Mittel, enthalten sein, fehlen bei Einsatz von Percarbonsäure als alleinigem Bleichmittel jedoch vorzugsweise ganz. Zusätzlich zu den konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein.

Zu den in den erfindungsgemäßen Mitteln, insbesondere wenn sie in flüssiger oder pastöser Form vorliegen, neben Wasser verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Mitteln vorzugsweise in Mengen nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den erfindungsgemäßen Mitteln in Mengen von vorzugsweise nicht über 20 Gew.-%, insbesondere von 1,2 Gew.-% bis 17 Gew.-%, enthalten. Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Erfindungsgemäße Textilwaschmittel können als optische Aufheller beispielsweise Derivate der Diaminostilbendisulfonsäure beziehungsweise deren Alkalimetallsalze enthalten, obgleich sie für den Einsatz als Colorwaschmittel vorzugsweise frei von optischen Aufhellern sind. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der MorpholinoGruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Insbesondere beim Einsatz in maschinellen Verfahren kann es von Vorteil sein, den Mitteln übliche Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinen und Bistearylethylendiamid bevorzugt.

In weiteren Ausführungsformen erfindungsgemäßer Mittel, insbesondere Wasch- oder Reinigungsmittel, werden die Proteasen beispielsweise mit einzelnen oder mehreren der folgenden Inhaltsstoffe kombiniert: nichtionische, anionische und/oder kationische Tenside, (gegebenenfalls weitere) Bleichmittel, Bleichaktivatoren, Bleichkatalysatoren, Builder und/oder Cobuilder, Säuren, alkalische Substanzen, Hydrotropen, Lösungsmittel, Verdicker, Sequestrierungsmittel, Elektrolyte, optische Aufheller, Vergrauungsinhibitoren, Korrosionsinhibitoren, insbesondere Silberschutzmittel (Silberkorrosionsinhibitoren), Desintegrationshilfsmittel, Soil-Release-Wirkstoffe, Farbtransfer (oder-Übertragungs)-Inhibitoren, Schauminhibitoren, Abrasivstoffe, Farbstoffe, Duftstoffe, Parfüms, antimikrobielle Wirkstoffe, UV-Schutzmittel bzw. -Absorbenzien, Antistatika, Perlglanzmitteln und Hautschutzmitteln, Enzyme wie beispielsweise Protease, Amylase, Cellulase, Hemicellulase, Mannanase, Tannase, Xylanase, Xanthanase, β-Glucosidase, Carrageenase, Oxidase, Oxidoreduktase, Pektin-abbauendes Enzym oder eine Lipase, Stabilisatoren, insbesondere Enzymstabilisatoren, und andere Komponenten, die aus dem Stand der Technik bekannt sind. In einer weiteren Ausführungsform der Erfindung ist ein erfindungsgemäßes Mittel somit dadurch gekennzeichnet, dass es mindestens eine weitere Komponente enthält, ausgewählt aus der Gruppe bestehend aus Tensiden, Buildern, Säuren, alkalischen Substanzen, Hydrotropen, Lösungsmitteln, Verdickungsmitteln, Bleichmitteln, Farbstoffen, Parfüms, Korrosionsinhibitoren, Sequestriermitteln, Elektrolyten, optischen Aufhellern, Vergrauungsinhibitoren, Silberkorrosionsinhibitoren, Farbübertragungsinhibitoren, Schauminhibitoren, Desintegrationshilfsmittel, Abrasivstoffen, UV-Absorbenzien, Lösungsmitteln, Antistatika, Perlglanzmitteln und Hautschutzmitteln.

Die zu wählenden Inhaltsstoffe wie auch die Bedingungen, unter denen das Mittel eingesetzt wird, wie beispielsweise Temperatur, pH-Wert, Ionenstärke, Redox-Verhältnisse oder mechanische Einflüsse, sollten für das jeweilige Reinigungsproblem optimiert sein. So liegen übliche Temperaturen für Wasch- und Reinigungsmittel in Bereichen von 10°C bei manuellen Mitteln über 40°C und 60°C bis hin zu 95° bei maschinellen Mitteln oder bei technischen Anwendungen. Da bei modernen Wasch- und Spülmaschinen die Temperatur meist stufenlos einstellbar ist, sind auch alle Zwischenstufen der Temperatur eingeschlossen. Vorzugsweise werden die Inhaltsstoffe der betreffenden Mittel aufeinander abgestimmt. Bevorzugt sind Synergien hinsichtlich der Reinigungsleistung. Besonders bevorzugt diesbezüglich sind Synergien, die sich aus dem Zusammenwirken der beiden Proteasen in dem erfindungsgemäßen Mittel mit mindestens einem oder mehreren weiteren Inhaltsstoff ergeben. In einer weiteren bevorzugten Ausführungsform sind diese Synergien in einem Temperaturbereich zwischen 20°C und 60°C vorhanden, da auch die in den erfindungsgemäßen Mitteln enthaltenen Proteasen in diesem Temperaturbereich katalytisch aktiv sind.

In einer weiteren Ausführungsform umfasst ein erfindungsgemäßes Mittel, insbesondere ein Wasch- oder Reinigungsmittel, ferner
- 5 Gew.-% bis 70 Gew.-%, insbesondere 5 Gew.-% bis 30 Gew.-% Tenside und/oder
- 10 Gew.-% bis 65 Gew.-%, insbesondere 12 Gew.-% bis 60 Gew. -% wasserlösliches oder wasserdispergierbares anorganisches Buildermaterial und/oder
- 0,5 Gew.-% bis 10 Gew.-%, insbesondere 1 Gew.-% bis 8 Gew.-%, wasserlösliche organische Buildersubstanzen und/oder
- 0,01 bis 15 Gew.-% feste anorganische und/oder organische Säuren beziehungsweise saure Salze und/oder
- 0,01 bis 5 Gew.-% Komplexbildner für Schwermetalle und/oder
- 0,01 bis 5 Gew.-% Vergrauungsinhibitor und/oder
- 0,01 bis 5 Gew. -% Farbübertragungsinhibitor und/oder
- 0,01 bis 5 Gew.-% Schauminhibitor.

Optional kann das Mittel ferner optische Aufheller umfassen, bevorzugt von 0,01 bis 5 Gew-%.

Die Herstellung erfindungsgemäßer fester Mittel bietet keine Schwierigkeiten und kann auf bekannte Weise, zum Beispiel durch Sprühtrocknen oder Granulation, erfolgen, wobei Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein einen Extrusionschritt aufweisendes Verfahren bevorzugt.

Zur Herstellung von erfindungsgemäßen Mitteln in Tablettenform, die einphasig oder mehrphasig, einfarbig oder mehrfarbig und insbesondere aus einer Schicht oder aus mehreren, insbesondere aus zwei Schichten bestehen können, geht man vorzugsweise derart vor, dass man alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN, vorzugsweise bei 60 bis 70 kN verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN, insbesondere bei 10 bis 15 kN durchgeführt. Man erhält so problemlos bruchfeste und dennoch unter Anwendungsbedingungen ausreichend schnell lösliche Tabletten mit Bruch- und Biegefestigkeiten von normalerweise 100 bis 200 N, bevorzugt jedoch über 150 N. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind. Ecken und Kanten sind vorteilhafterweise abgerundet. Runde Tabletten weisen vorzugsweise einen Durchmesser von 30 mm bis 40 mm auf. Insbesondere die Größe von eckig oder quaderförmig gestalteten Tabletten, welche überwiegend über die Dosiervorrichtung beispielsweise der Geschirrspülmaschine eingebracht werden, ist abhängig von der Geometrie und dem Volumen dieser Dosiervorrichtung. Beispielhaft bevorzugte Ausführungsformen weisen eine Grundfläche von (20 bis 30 mm) x (34 bis 40 mm), insbesondere von 26x36 mm oder von 24x38 mm auf.

Flüssige beziehungsweise pastöse erfindungsgemäße Mittel in Form von übliche Lösungsmittel enthaltenden Lösungen werden in der Regel durch einfaches Mischen der Inhaltsstoffe, die in Substanz oder als Lösung in einen automatischen Mischer gegeben werden können, hergestellt. Ausführungsformen der vorliegenden Erfindung umfassen somit alle derartigen festen, pulverförmigen, flüssigen, gelförmigen oder pastösen Darreichungsformen der Mittel, die gegebenenfalls auch aus mehreren Phasen bestehen können sowie in komprimierter oder nicht komprimierter Form vorliegen können. Eine weitere Ausführungsform der Erfindung stellen daher Mittel dar, die dadurch gekennzeichnet sind, dass sie als Einkomponentensystem vorliegen. Solche Mittel bestehen bevorzugt aus einer Phase. Selbstverständlich können erfindungsgemäße Mittel aber auch aus mehreren Phasen bestehen. In einer weiteren Ausführungsform der Erfindung ist das Wasch- oder Reinigungsmittel daher dadurch gekennzeichnet sind, dass es in mehrere Komponenten aufgeteilt ist.

Zu den erfindungsgemäßen festen Darreichungsformen zählen ferner Extrudate, Granulate, Tabletten oder Pouches, die sowohl in Großgebinden als auch portionsweise abgepackt vorliegen können. Alternativ liegt das Mittel als rieselfähiges Pulver vor, insbesondere mit einem Schüttgewicht von 300 g/l bis 1200 g/l, insbesondere 500 g/l bis 900 g/l oder 600 g/l bis 850 g/l.

In einer weiteren Ausführungsform der Erfindung liegt das Mittel, insbesondere das Wasch- oder Reinigungsmittel, in flüssiger, gelförmiger oder pastöser Form vor, insbesondere in Form eines nicht-wässrigen Flüssigwaschmittels oder einer nicht-wässrigen Paste oder in Form eines wässrigen Flüssigwaschmittels oder einer wasserhaltigen Paste.

Das erfindungsgemäße Mittel, insbesondere Wasch- oder Reinigungsmittel, kann in einem Behältnis, vorzugsweise einem luftdurchlässigen Behältnis, verpackt sein, aus dem es kurz vor Gebrauch oder während des Waschvorgangs freigesetzt wird. Insbesondere kann ferner mindestens eine, bevorzugt beide der in dem Mittel enthaltenen Proteasen und/oder weitere Inhaltsstoffe des Mittels mit einer bei Raumtemperatur oder bei Abwesenheit von Wasser für das Enzym/die Enzyme undurchlässigen Substanz umhüllt sein, welche unter Anwendungsbedingungen des Mittels durchlässig für das Enzym/die Enzyme wird. Eine solche Ausführungsform der Erfindung ist somit dadurch gekennzeichnet, dass mindestens eine Protease mit einer bei Raumtemperatur oder bei Abwesenheit von Wasser für die Protease undurchlässigen Substanz umhüllt ist.

Erfindungsgemäße Mittel können ausschließlich die vorstehend beschriebenen mindestens zwei Proteasen enthalten. Alternativ können sie auch weitere Proteasen oder andere Enzyme in einer für die Wirksamkeit des Mittels zweckmäßigen Konzentration enthalten. Einen weiteren Gegenstand der Erfindung stellen somit Mittel dar, die ferner eines oder mehrere weitere Enzyme umfassen, wobei prinzipiell alle im Stand der Technik für diese Zwecke etablierten Enzyme einsetzbar sind. Als weitere Enzyme bevorzugt einsetzbar sind alle Enzyme, die in dem erfindungsgemäßen Mittel eine katalytische Aktivität entfalten können, insbesondere Proteasen, Amylasen, Cellulasen, Hemicellulasen, Mannanasen, Tannasen, Xylanasen, Xanthanasen, β-Glucosidasen, Carrageenasen, Oxidasen, Oxidoreduktasen, Pektin-abbauende Enzyme (Pektinasen) oder Lipasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden. Erfindungsgemäße Mittel enthalten Enzyme vorzugsweise in Gesamtmengen von 1 x 10⁻⁸ bis 5 Gewichts-Prozent bezogen auf aktives Protein. Bevorzugt sind die Enzyme von 0,00001 bis 5 Gew.-%, weiter bevorzugt von 0,0001 bis 2,5 Gew.-%, noch weiter bevorzugt von 0,0001 bis 1 Gew.-% und besonders bevorzugt von 0,0001 bis 0,072 Gew.-% in erfindungsgemäßen Mitteln enthalten, wobei jedes enthaltene Enzym in den genannten Mengenverhältnissen vorliegen kann.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Besonders bevorzugt unterstützen die weiteren Enzyme die Wirkung des Mittels, beispielsweise die Reinigungsleistung eines Wasch- oder Reinigungsmittels, hinsichtlich bestimmter Anschmutzungen oder Flecken. Besonders bevorzugt zeigen die Enzyme synergistische Effekte hinsichtlich ihrer Wirkung gegenüber bestimmter Anschmutzungen oder Flecken, d.h. die in der Mittelzusammensetzung enthaltenen Enzyme unterstützen sich in ihrer Reinigungsleistung gegenseitig. Synergistische Effekte können nicht nur zwischen verschiedenen Enzymen, sondern auch zwischen einem oder mehreren Enzymen und weiteren Inhaltsstoffen des erfindungsgemäßen Mittels auftreten. In einer weiteren bevorzugten Ausführungsform der Erfindung ist das erfindungsgemäße Mittel somit dadurch gekennzeichnet, dass es mindestens ein weiteres Enzym enthält, welches eine Protease, Amylase, Cellulase, Hemicellulase, Mannanase, Tannase, Xylanase, Xanthanase, β-Glucosidase, Carrageenase, Oxidase, Oxidoreduktase, Pektin-abbauendes Enzym oder eine Lipase ist.

Bei dem Vergleich der Leistungen zweier Enzyme, ist zwischen proteingleichem und aktivitätsgleichem Einsatz zu unterscheiden. Insbesondere bei gentechnisch erhaltenen, weitgehend nebenaktivitätsfreien Präparationen ist der proteingleiche Einsatz angebracht. Denn damit ist eine Aussage darüber möglich, ob dieselben Proteinmengen - beispielsweise als Maß für den Ertrag einer fermentativen Produktion - zu vergleichbaren Ergebnissen führen. Klaffen die jeweiligen Verhältnisse von Aktivsubstanz zu Gesamtprotein (die Werte der spezifischen Aktivität) auseinander, so ist ein aktivitätsgleicher Vergleich zu empfehlen, weil hierüber die jeweiligen enzymatischen Eigenschaften verglichen werden. Generell gilt, dass eine niedrige spezifische Aktivität durch Zugabe einer größeren Proteinmenge ausgeglichen werden kann. Hierbei handelt es sich letztlich um eine ökonomische Erwägung.

Die Proteaseaktivität in derartigen Mitteln kann nach der in Tenside, Band 7 (1970), S. 125-132 beschriebenen Methode ermittelt werden. Sie wird entsprechend in PE (Protease-Einheiten) angegeben.

Die in erfindungsgemäßen Mitteln eingesetzten Enzyme stammen entweder ursprünglich aus Mikroorganismen, etwa der Gattungen Bacillus, Streptomyces, Humicola, oder Pseudomonas, und/oder werden nach an sich bekannten biotechnologischen Verfahren durch geeignete Mikroorganismen produziert, beispielsweise durch transgene Expressionswirte der Gattungen Bacillus oder durch filamentöse Fungi.

Einen eigenen Erfindungsgegenstand stellt die Verwendung eines oben beschriebenen erfindungsgemäßen Mittels zur Entfernung von Protease-sensitiven Anschmutzungen auf Textilien oder harten Oberflächen, d.h. zur Reinigung von Textilien oder von harten Oberflächen, dar.

Denn erfindungsgemäße Mittel können, insbesondere entsprechend den oben beschriebenen Eigenschaften, dazu verwendet werden, um von Textilien oder von harten Oberflächen proteinhaltige Verunreinigungen zu beseitigen. Ausführungsformen stellen beispielsweise die Handwäsche, die manuelle Entfernung von Flecken von Textilien oder von harten Oberflächen oder die Verwendung im Zusammenhang mit einem maschinellen Verfahren dar.

In einer bevorzugten Ausführungsform dieser Verwendung werden die betreffenden erfindungsgemäßen Mittel, vorzugsweise Wasch- beziehungsweise Reinigungsmittel, nach einer der oben ausgeführten Ausführungsformen bereitgestellt.

Einen weiteren eigenen Erfindungsgegenstand stellen Verfahren zur Reinigung von Textilien oder von harten Oberflächen dar, bei denen wenigstens in einem der Verfahrensschritte ein erfindungsgemäßes Mittel verwendet wird. Das Verfahren zur Reinigung von Textilien oder harten Oberflächen ist demnach dadurch gekennzeichnet, dass in mindestens einem Verfahrensschritt ein erfindungsgemäßes Mittel angewendet ist.

Hierunter fallen sowohl manuelle als auch maschinelle Verfahren, wobei maschinelle Verfahren aufgrund ihrer präziseren Steuerbarkeit, was beispielsweise die eingesetzten Mengen und Einwirkzeiten angeht, bevorzugt sind.

Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung dieses Mittels behandelt wird. Das gleiche gilt für Verfahren zur Reinigung von allen anderen Materialien als Textilien, welche unter dem Begriff harte Oberflächen zusammengefasst werden. Alle denkbaren Wasch- oder Reinigungsverfahren können in wenigstens einem der Verfahrensschritte um ein erfindungsgemäßes Mittel bereichert werden, und stellen dann Ausführungsformen der vorliegenden Erfindung dar.

Da bevorzugte erfindungsgemäße Proteasekombinationen natürlicherweise bereits eine proteinauflösende Aktivität besitzen und diese auch in Medien entfalten, die sonst keine Reinigungskraft besitzen, wie beispielsweise in bloßem Puffer, kann ein einzelner Teilschritt eines solchen Verfahrens zur Reinigung, insbesondere zur maschinellen Reinigung, von Textilien oder harten Oberflächen darin bestehen, dass gewünschten falls neben stabilisierenden Verbindungen, Salzen oder Puffersubstanzen als einzige reinigungsaktive Komponente ein erfindungsgemäße Enzymmischung aufgebracht wird. Dies stellt eine besonders vereinfachte Ausführungsform der vorliegenden Erfindung dar.

Einen weiteren Gegenstand der Erfindung stellen Verfahren zur Reinigung von Textilien oder harten Oberflächen dar, die dadurch gekennzeichnet sind, dass in mindestens einem Verfahrensschritt eine erste Protease und mindestens eine zweite Protease proteolytisch aktiv sind, wobei
a) die erste Protease einer Aminosäuresequenz entspricht zu 100% identisch ist und
b) die zweite Protease ausgewählt ist unter Proteasen, die einer Aminosäuresequenz entsprechen, die zu der in SEQ ID NO.6 oder SEQ ID NO.7 angegebenen Aminosäuresequenz mindestens zu 80% und zunehmend bevorzugt zu mindestens 82,5%, 85%, 87,5%, 90%, 91%, 92%, 93%, 94%, 95%, 96%, 97%, 98%, 99%, 99,5% und ganz besonders bevorzugt zu 100% identisch ist und
c) die gegebenenfalls vorhandenen weiteren Proteasen beliebige, von der ersten und der zweiten Protease verschiedene Proteasen sein können, vorzugsweise jedoch ausgewählt sind unter Proteasen, die eine Aminosäuresequenz umfassen, die zu einer der in SEQ ID NO.2 bis SEQ ID NO.7 angegebenen Aminosäuresequenzen mindestens zu 80% und zunehmend bevorzugt zu mindestens 82,5%, 85%, 87,5%, 90%, 91%, 92%, 93%, 94%, 95%, 96%, 97%, 98%, 99%, 99,5% und ganz besonders bevorzugt zu 100% identisch ist.

Bevorzugt werden die Proteasen in einer Menge von 40 µg bis 4 g, vorzugsweise von 50 µg bis 3 g, besonders bevorzugt von 100 µg bis 2 g und ganz besonders bevorzugt von 200 µg bis 1 g pro Anwendung eingesetzt. In den dargestellten Mengenangaben sind die erste Protease, die zweite Protease und die gegebenenfalls vorhandenen weiteren Proteasen enthalten.

Weitere Ausführungsformen dieses Erfindungsgegenstandes stellen Verfahren zur Behandlung von Textilrohstoffen oder zur Textilpflege dar, bei denen in wenigstens einem der Verfahrensschritte eine erfindungsgemäße Proteasekombination aktiv wird.

Hierunter sind Verfahren für Textilrohstoffe, Fasern oder Textilien mit natürlichen Bestandteilen bevorzugt, und ganz besonders für solche mit Wolle oder Seide.

Es kann sich dabei beispielsweise um Verfahren handeln, in denen Materialien zur Verarbeitung in Textilien vorbereitet werden, etwa zur Antifilzausrüstung, oder beispielsweise um Verfahren, welche die Reinigung getragener Textilien um eine pflegende Komponente bereichern. Wegen der oben beschriebenen Wirkung von Proteasen auf natürliche, proteinhaltige Rohstoffe handelt es sich in bevorzugten Ausführungsformen um Verfahren zur Behandlung von Textilrohstoffen, Fasern oder Textilien mit natürlichen Bestandteilen, insbesondere mit Wolle oder Seide.

Ein weiterer Gegenstand der vorliegenden Erfindung ist die Verwendung gemäß Anspruch 8.

Denn erfindungsgemäße Kombinationen von Proteasen können, insbesondere auf Grund der vorstehend beschriebenen Eigenschaften, dazu verwendet werden, um von Textilien oder von harten Oberflächen proteinhaltige Verunreinigungen zu beseitigen. Ausführungsformen stellen beispielsweise die Handwäsche, die manuelle Entfernung von Flecken von Textilien oder von harten Oberflächen oder die Verwendung im Zusammenhang mit einem maschinellen Verfahren dar. In einer bevorzugten Ausführungsform dieser Verwendung werden die betreffenden erfindungsgemäßen Kombinationen von Proteasen nach einer der vorstehend ausgeführten Rezepturen bereitgestellt.

Ein weiterer Gegenstand der vorliegenden Erfindung ist die Verwendung einer ersten Protease und einer zweiten Protease gemäß Anspruch 7.

Alle Sachverhalte, Gegenstände und Ausführungsformen, die für erfindungsgemäße Wasch- oder Reinigungsmittel beschrieben sind, sind auch auf die genannten Verwendungen anwendbar. Daher wird an dieser Stelle ausdrücklich auf die Offenbarung an entsprechender Stelle verwiesen, mit dem Hinweis, dass diese Offenbarung auch für die vorstehenden erfindungsgemäßen Verwendungen gilt.

Die folgenden Beispiele erläutern die Erfindung, ohne sie jedoch darauf einzuschränken:
Beispiel 1: Synergistische Leistung der Proteasen gemäß SEQ ID NO.1 und SEQ ID NO.6.

Es wurden vier Versuche unter folgenden Bedingungen vorgenommen:

### 1. Verwendete Testanschmutzungen:

∘ Blut-Milch/Tusche auf Baumwolle: Produkt Nr. C5 von CFT B.V. Vlaardingen, Holland
∘ Blut auf Baumwolle: Produkt Nr. CS1 von CFT B.V. Vlaardingen, Holland
∘ Schokolade-Milch/Tusche auf Baumwolle: Produkt Nr. C3 von CFT B.V. Vlaardingen, Holland
∘ Voll Ei/Tusche auf Baumwolle: Produkt Nr. CS37 von CFT B.V. Vlaardingen, Holland
∘ Equest Napolitana Tomato Puree von erhältlich von der Firma Warwick Equest Ltd Unit 55, Consett Business Park, Consett, County Durham,
∘ Equest Grass & Mud von erhältlich von der Firma Warwick Equest Ltd Unit 55, Consett Business Park, Consett, County Durham,
∘ Equest French*s Squeezy Mus-tard von erhältlich von der Firma Warwick Equest Ltd Unit 55, Consett Business Park, Consett, County Durham,

Die Waschversuche wurden in einer Waschmaschine der Fa. Miele (Miele Novotronic W308) mit 3,5 kg Ballastwäsche durchgeführt.
Insgesamt wurde sechsmal gewaschen.
Das Flottenverhältnis betrug 1:4.
Es wurde wie folgt dosiert: 4 g des Mittels / 1000 ml
Die Wasserhärte lag bei 16°dH
Gewaschen wurde bei zwei verschiedenen Temperaturen:
   a) bei einer Waschtemperatur von 40°C (Programm 6) über 60 Minuten
   b) bei einer Waschtemperatur von 20°C (Programm 30) über 60 Minuten

Einzelne Proteasen bzw. Proteasegemische wurden in ein westeuropäisches Premiumwaschmittel mit folgender Zusammensetzung eingearbeitet (Inhaltsstoffe in Gewichtsprozent, jeweils bezogen auf das gesamte Mittel): 0,3-0,5% Xanthan, 0,2-0,4% Anti-Schaummittel, 6-7% Glycerin, 0,3-0,5% Ethanol, 4-7% FAEOS (Fettalkoholethersulfat), 24-28% nichtionische Tenside, 1 % Borsäure, 1 - 2% Natriumcitrat (Dihydrat), 2-4% Soda, 14-16% Kokosnuss-Fettsäuren, 0,5% HEDP (1 - Hydroxyethan-(1,1-di- phosphonsäure)), 0-0,4% PVP (Polyvinylpyrrolidon), 0-0,05% optischer Aufheller, 0-0,001 % Farbstoff, Rest demineralisiertes Wasser.

Die Geamtmenge an Protease enthalten in einer Waschladung war 25mg bei Einsatz von nur einer Protease gemäss SEQ ID NO.1, 6 oder 7. Bei Einsatz eines Proteasepaares wurden jeweils 12,5 mg der Protease gemäss SEQ ID NO.1 und 25 mg der Protease gemäss SEQ ID NO.6 oder SEQ ID NO.7 pro Waschladung hinzugegeben.

Die folgenden Tabellen zeigen die relative proteolytische Aktivität jeweils eines Paars von Proteasen oder eines Paars einer Protease und eines Proteasegemisches.
"nd" steht hierbei für "nicht bestimmt".
I. Protease (SEQ ID NO.1) verringert bei einer Waschtemperatur von 20°C Anschmutzungen mehrheitlich in geringerem Maße als Protease (SEQ ID NO.6):

| Anschmutzung | SEQ 6 - SEQ 1 |
|---|---|
| CFT C05 | -8,1 |
| CFT C03 | -9,2 |
| CFT CS01 | nd |
| CFT CS37 | -4,4 |
| Equest Napolitana Tomato Puree | nd |
| Equest Grass & Mud | -0,5 |
| Equest French*s Squeezy Mustard | 0,4 |

II. Protease (SEQ ID NO.1) verringert bei einer Waschtemperatur von 20°C Anschmutzungen mehrheitlich in geringerem Maße als Protease (SEQ ID NO.7):

| Anschmutzung | SEQ 7 - SEQ 1 |
|---|---|
| CFT C05 | -6,0 |
| CFT C03 | -8,2 |
| CFT CS01 | -0,8 |
| CFT CS37 | -4,8 |
| Equest Napolitana Tomato Puree | -0,8 |
| Equest Grass & Mud | nd |
| Equest French*s Squeezy Mustard | nd |

III. Ein erfindungsgemäßes Gemisch aus Protease (SEQ ID NO.1) und Protease (SEQ ID NO.6) verringert bei einer Waschtemperatur von 20°C Anschmutzungen mehrheitlich in höherem Maße als Protease (SEQ ID NO.6) allein:

| Anschmutzung | SEQ 6 - SEQ 6/SEQ 1 |
|---|---|
| CFT C05 | 0,8 |
| CFT C03 | 0,6 |
| CFT CS01 | nd |
| CFT CS37 | 1,5 |
| Equest Napolitana Tomato Puree | nd |
| Equest Grass & Mud | 1,0 |
| Equest French*s Squeezy Mustard | 0,8 |

IV. Ein erfindungsgemäßes Gemisch aus Protease (SEQ ID NO.7) und Protease (SEQ ID NO.1) verringert bei einer Waschtemperatur von 20°C Anschmutzungen mehrheitlich in höherem Maße als Protease (SEQ ID NO.7) allein:

| Anschmutzung | SEQ 7- SEQ 7/SEQ 1 |
|---|---|
| CFT C05 | 0,4 |
| CFT C03 | 1,4 |
| CFT CS01 | 2,2 |
| CFT CS37 | 1,9 |
| Equest Napolitana Tomato Puree | 1,9 |
| Equest Grass & Mud | nd |
| Equest French*s Squeezy Mustard | nd |

Die Versuche III und IV dokumentieren die überlegene synergistische Wirkung eines erfindungsgemäßen Mittels, bzw. eines erfindungsgemäßen Enzymgemischs.

## Patentansprüche

1. Wasch- oder Reinigungsmittel, enthaltend eine erste Protease und mindestens eine zweite Protease, **dadurch gekennzeichnet, dass** a) die erste Protease der in SEQ ID NO.1 angegebenen Aminosäuresequenz entspricht und b) die zweite Protease ausgewählt ist unter Proteasen, die der in SEQ ID NO.6 oder SEQ ID NO.7 angegebenen Aminosäuresequenz entsprechen oder mindestens zu 80% und zunehmend bevorzugt zu mindestens 82,5%, 85%, 87,5%, 90%, 91%, 92%, 93%, 94%, 95%, 96%, 97%, 98%, 99%, 99,5% und ganz besonders bevorzugt zu 100% zu dieser identisch ist.

2. Mittel nach Anspruch 1, **dadurch gekennzeichnet, dass** das Mittel zusätzlich zu der ersten Protease und der zweiten Protease noch eine oder mehrere weitere Proteasen enthält, vorzugsweise solche, die ausgewählt sind unter Proteasen, die einer der in SEQ ID NO.2 bis SEQ ID NO.7 angegebenen Aminosäuresequenzen entsprechen oder mindestens zu 80% und zunehmend bevorzugt zu mindestens 82,5%, 85%, 87,5%, 90%, 91%, 92%, 93%, 94%, 95%, 96%, 97%, 98%, 99%, 99,5% und ganz besonders bevorzugt zu 100% zu dieser identisch ist.

3. Mittel nach einem der vorhergehenden Ansprüche, **dadurch gekennzeichnet, dass** das Mittel die mindestens zwei Proteasen insbesondere in Gesamtmengen von 0,05 bis 5 Gew.-%, vorzugsweise 0,05 bis 2 Gew.-%, bezogen auf die Gesamtmenge des Mittels enthält.

4. Mittel nach einem der vorhergehenden Ansprüche, **dadurch gekennzeichnet, dass** das Mittel die mindestens zwei Proteasen insbesondere insbesondere in Gesamtmengen von 0,05 bis 15 Gew.-%, vorzugsweise 0,05 bis 10 Gew.- %, bezogen auf aktives Protein enthält.

5. Mittel nach einem der Ansprüche 1 bis 4, **dadurch gekennzeichnet, dass** es mindestens eine weitere Komponente enthält, ausgewählt aus der Gruppe bestehend aus Tensiden, Buildern, Säuren, alkalischen Substanzen, Hydrotropen, Lösungsmitteln, Verdickungsmitteln, Bleichmitteln, Farbstoffen, Parfums, Korrosionsinhibitoren, Sequestriermitteln, Elektrolyten, optischen Aufhellern, Vergrauungsinhibitoren, Silberkorrosionsinhibitoren, Farbübertragungsinhibitoren, Schauminhibitoren, Desintegrationshilfsmittel, Abrasivstoffen, UV-Absorbenzien, Lösungsmitteln, Antistatika, Perlglanzmitteln und Hautschutzmitteln.

6. Verfahren zur Reinigung von Textilien oder harten Oberflächen, **dadurch gekennzeichnet, dass** in mindestens einem Verfahrensschritt eine erste Protease und mindestens eine zweite Protease proteolytisch aktiv sind, wobei a) die erste Protease der in SEQ ID NO.1 angegebenen Aminosäuresequenz entspricht, und b) die zweite Protease ausgewählt ist unter Proteasen, die der in SEQ ID NO.6 oder SEQ ID NO.7 angegebenen Aminosäuresequenz entsprechen oder mindestens zu 80% und zunehmend bevorzugt zu mindestens 82,5%, 85%, 87,5%, 90%, 91%, 92%, 93%, 94%, 95%, 96%, 97%, 98%, 99%, 99,5% und ganz besonders bevorzugt zu 100% zu dieser identisch ist.

7. Verwendung einer ersten Protease und mindestens einer zweiten Protease zur Reinigung von Textilien oder harten Oberflächen dar, **dadurch gekennzeichnet, dass** a) die erste Protease der in SEQ ID NO.1 angegebenen Aminosäuresequenz entspricht, und b) die zweite Protease ausgewählt ist unter Proteasen, die der in SEQ ID NO.6 oder SEQ ID NO.7 angegebenen Aminosäuresequenz entsprechen oder mindestens zu 80% und zunehmend bevorzugt zu mindestens 82,5%, 85%, 87,5%, 90%, 91%, 92%, 93%, 94%, 95%, 96%, 97%, 98%, 99%, 99,5% und ganz besonders bevorzugt zu 100% zu dieser identisch ist.

8. Verwendung einer Protease, die eine Aminosäuresequenz umfasst, die der in SEQ ID NO.1 angegebenen Aminosäuresequenz entspricht, als Booster zur Verbesserung der proteolytischen Wirkung anderer Proteasen oder Proteasegemische.

## Claims

1. Washing or cleaning agent containing a first protease and at least one second protease, **characterized in that**
a) the first protease corresponds to the amino acid sequence shown in SEQ ID NO.1 and
b) the second protease is selected from proteases which correspond to the amino acid sequence shown in SEQ ID NO. 6 or SEQ ID NO. 7 or are at least 80%, and more preferably at least 82.5%, 85%, 87.5%, 90%, 91%, 92%, 93%, 94%, 95%, 96%, 97%, 98%, 99%, 99.5%, and most preferably 100% identical thereto.

2. The agent according to claim 1, **characterized in that** the agent, in addition to the first protease and the second protease, also contains one or more further proteases, preferably those which are selected from proteases which correspond to one of the amino acid sequences shown in SEQ ID NO.2 to SEQ ID NO.7 or are at least 80%, and more preferably at least 82.5%, 85%, 87.5%, 90%, 91%, 92%, 93%, 94%, 95%, 96%, 97%, 98%, 99%, 99.5%, and most preferably 100% identical thereto.

3. The agent according to one of the preceding claims, **characterized in that** the agent contains the at least two proteases, in particular in total amounts of from 0.05 to 5 wt.%, preferably from 0.05 to 2 wt.%, based on the total amount of the agent.

4. The agent according to one of the preceding claims, **characterized in that** the agent contains the at least two proteases, in particular in total amounts of from 0.05 to 15 wt.%, preferably from 0.05 to 10 wt.%, based on active protein.

5. The agent according to one of claims 1 to 4, **characterized in that** it contains at least one further component selected from the group consisting of surfactants, builders, acids, alkaline substances, hydrotropic substances, solvents, thickening agents, bleaching agents, dyes, perfumes, corrosion inhibitors, sequestering agents, electrolytes, optical brighteners, graying inhibitors, silver corrosion inhibitors, dye transfer inhibitors, suds suppressors, disintegration auxiliaries, abrasives, UV absorbents, solvents, antistatic agents, pearlescing agents and skin protection agents.

6. A method for cleaning textiles or hard surfaces, **characterized in that**, in at least one method step, a first protease and at least one second protease are proteolytically active,
a) the first protease corresponding to the amino acid sequence shown in SEQ ID NO.1, and
b) the second protease being selected from proteases which correspond to the amino acid sequence shown in SEQ ID NO. 6 or SEQ ID NO. 7 or are at least 80%, and more preferably at least 82.5%, 85%, 87.5%, 90%, 91%, 92%, 93%, 94%, 95%, 96%, 97%, 98%, 99%, 99.5%, and most preferably 100% identical thereto.

7. The use of a first protease and at least one second protease for cleaning textiles or hard surfaces, **characterized in that**
a) the first protease corresponds to the amino acid sequence shown in SEQ ID NO.1 and
b) the second protease is selected from proteases which correspond to the amino acid sequence shown in SEQ ID NO. 6 or SEQ ID NO. 7 or are at least 80%, and more preferably at least 82.5%, 85%, 87.5%, 90%, 91%, 92%, 93%, 94%, 95%, 96%, 97%, 98%, 99%, 99.5%, and most preferably 100% identical thereto.

8. The use of a protease which comprises an amino acid sequence which corresponds to the amino acid sequence shown in SEQ ID NO.1 as a booster for improving the proteolytic effect of other proteases or protease mixtures.

## Revendications

1. Composition de lavage ou de nettoyage comprenant une première protéase et au moins une seconde protéase, **caractérisée en ce que**
a) la première protéase correspond à la séquence d'acides aminés indiquée dans SEQ ID N°1 et
b) la seconde protéase est choisie parmi les protéases qui correspond à la séquence d'acides aminés indiquée dans SEQ ID N° 6 ou SEQ ID N° 7 ou sont identique à raison d'au moins 80 % et de manière préférée de façon croissante à raison d'au moins 82,5 %, 85 %, 87,5 %, 90 %, 91 %, 92 %, 93 %, 94 %, 95 %, 96 %, 97 %, 98 %, 99 %, 99,5 % et de manière tout particulièrement préférée à raison de 100 % à celle-ci.

2. Agent selon la revendication 1, **caractérisé en ce que** l'agent contient, en plus de la première protéase et de la seconde protéase, une ou plusieurs protéases supplémentaires, de préférence celles qui sont choisies parmi les protéases correspondent à l'une des séquences d'acides aminés indiquée dans SEQ ID N° 2 à SEQ ID N° 7 ou sont identique à raison d'au moins 80 %, et de manière préférée de façon croissante à raison d'au moins 82,5 %, 85 %, 87,5%, 90 %, 91 %, 92 %, 93 %, 94 %, 95 %, 96 %, 97 %, 98 %, 99 %, 99,5 % et de manière tout particulièrement préférée à raison de 100 % à celle-ci.

3. Agent selon l'une quelconque des revendications précédentes, **caractérisée en ce que** l'agent contient les au moins deux protéases, en particulier dans des quantités totales de 0,05 à 5 % en poids, de préférence de 0,05 à 2 % en poids, par rapport à la quantité totale de l'agent.

4. Agent selon l'une quelconque des revendications précédentes, **caractérisée en ce que** l'agent contient les au moins deux protéases, en particulier dans des quantités totales de 0,05 à 15 % en poids, de préférence de 0,05 à 10 % en poids, par rapport à une protéine active.

5. Agent selon l'une quelconque des revendications 1 à 4, **caractérisée en ce qu'**il contient au moins un autre composant choisi dans le groupe constitué des tensioactifs, des adjuvants, des acides, des substances alcalines, des hydrotropes, des solvants, des épaississants, des agents de blanchiment, des colorants, des parfums, des inhibiteurs de corrosion, des agents de séquestration, des électrolytes, des azurants optiques, des inhibiteurs de grisonnement, des inhibiteurs de corrosion à l'argent, des inhibiteurs de transfert de couleur, des inhibiteurs de mousse, des agents de désintégration, des substances abrasives, des absorbeurs d'UV, des solvants, des antistatiques, des agents nacrants et des agents de protection de la peau.

6. Procédé pour le nettoyage de textiles ou de surfaces dures, **caractérisé en ce que**, dans au moins une étape de procédé, une première protéase et au moins une seconde protéase sont protéolytiquement actives, dans lequel
a) la première protéase correspond à la séquence d'acides aminés indiquée dans SEQ ID N° 1 et
b) la seconde protéase est choisie parmi les protéases qui correspondent à la séquence d'acides aminés indiquée dans SEQ ID N° 6 ou SEQ ID N° 7 ou sont identique à raison d'au moins 80 % et de manière préférée de manière croissante à raison d'au moins 82,5 %, 85 %, 87,5 %, 90 %, 91 %, 92 %, 93 %, 94 %, 95 %, 96 %, 97 %, 98 %, 99 %, 99,5 % et de manière tout particulièrement préférée à raison de 100 % à celle-ci.

7. Utilisation d'une première protéase et d'au moins une seconde protéase pour le nettoyage de textiles ou de surfaces dures, **caractérisée en ce que**
a) la première protéase correspond à la séquence d'acides aminés indiquée dans SEQ ID N° 1et
b) la seconde protéase est choisie parmi les protéases qui correspondent à la séquence d'acides aminés indiquée dans SEQ ID N°6 ou SEQ ID N° 7 ou sont identique à raison d'au moins 80 % et de manière préférée de manière croissante à raison d'au moins 82,5 %, 85 %, 87,5 %, 90 %, 91 %, 92 %, 93 %, 94 %, 95 %, 96 %, 97 %, 98 %, 99 %, 99,5 % et de manière tout particulièrement préférée à raison de 100 % à celle-ci.

8. Utilisation d'une protéase qui comprend une séquence d'acides aminés correspondant à la séquence d'acides aminés indiquée dans SEQ ID N° 1 en tant que renforçateur pour améliorer l'action protéolytique d'autres protéases ou de mélanges de protéases.


### SEQUENCE LISTING

<110> Henkel AG & Co. KGaA
<120> Wasch- und Reinigungsmittel, enthaltend mindestens zwei Proteasen
<130> PT032398PCT
<150> 102014223296.1
   <151> 2014-11-14
<160> 7
<170> PatentIn version 3.5
<210> 1
   <211> 274
   <212> PRT
   <213> Bacillus Licheniformis
<400> 1
   Ala Gln Thr Val Pro Tyr Gly Ile Pro Leu Ile Lys Ala Asp Lys Val
   1               5                   10                  15
   Gln Ala Gln Gly Phe Lys Gly Ala Asn Val Lys Val Ala Val Leu Asp
               20                  25                  30
   Thr Gly Ile Gln Ala Ser His Pro Asp Leu Asn Val Val Gly Gly Ala
           35                  40                  45
   Ser Phe Val Ala Gly Glu Ala Tyr Asn Thr Asp Gly Asn Gly His Gly
       50                  55                  60
   Thr His Val Ala Gly Thr Val Ala Ala Leu Asp Asn Thr Thr Gly Val
   65                  70                  75                  80
   Leu Gly Val Ala Pro Ser Val Ser Leu Tyr Ala Val Lys Val Leu Asn
                   85                  90                  95
   Ser Ser Gly Ser Gly Ser Tyr Ser Gly Ile Val Ser Gly Ile Glu Trp
               100                 105                 110
   Ala Thr Thr Asn Gly Met Asp Val Ile Asn Met Ser Leu Gly Gly Ala
           115                 120                 125
   Ser Gly Ser Thr Ala Met Lys Gln Ala Val Asp Asn Ala Tyr Ala Arg
       130                 135                 140
   Gly Val Val Val Val Ala Ala Ala Gly Asn Ser Gly Ser Ser Gly Asn
   145                 150                 155                 160
   Thr Asn Thr Ile Gly Tyr Pro Ala Lys Tyr Asp Ser Val Ile Ala Val
   165 170 175
   Gly Ala Val Asp Ser Asn Ser Asn Arg Ala Ser Phe Ser Ser Val Gly
   180 185 190
   Ala Glu Leu Glu Val Met Ala Pro Gly Ala Gly Val Tyr Ser Thr Tyr
   195 200 205
   Pro Thr Asn Thr Tyr Ala Thr Leu Asn Gly Thr Ser Met Ala Ser Pro
   210 215 220
   His Val Ala Gly Ala Ala Ala Leu Ile Leu Ser Lys His Pro Asn Leu
   225 230 235 240
   Ser Ala Ser Gln Val Arg Asn Arg Leu Ser Ser Thr Ala Thr Tyr Leu
   245 250 255
   Gly Ser Ser Phe Tyr Tyr Gly Lys Gly Leu Ile Asn Val Glu Ala Ala
   260 265 270
   Ala Gln
<210> 2
   <211> 379
   <212> PRT
   <213> Bacillus Licheniformis
<400> 2
   Met Met Arg Lys Lys Ser Phe Trp Leu Gly Met Leu Thr Ala Phe Met
   1 5 10 15
   Leu Val Phe Thr Met Ala Phe Ser Asp Ser Ala Ser Ala Ala Gln Pro
   20 25 30
   Ala Lys Asn Val Glu Lys Asp Tyr Ile Val Gly Phe Lys Ser Gly Val
   35 40 45
   Lys Thr Ala Ser Val Lys Lys Asp Ile Ile Lys Glu Ser Gly Gly Lys
   50 55 60
   Val Asp Lys Gln Phe Arg Ile Ile Asn Ala Ala Lys Ala Lys Leu Asp
   65 70 75 80
   Lys Glu Ala Leu Lys Glu Val Lys Asn Asp Pro Asp Val Ala Tyr Val
   85 90 95
   Glu Glu Asp His Val Ala His Ala Leu Ala Gln Thr Val Pro Tyr Gly
   100 105 110
   Ile Pro Leu Ile Lys Ala Asp Lys Val Gln Ala Gln Gly Phe Lys Gly
   115 120 125
   Ala Asn Val Lys Val Ala Val Leu Asp Thr Gly Ile Gln Ala Ser His
   130 135 140
   Pro Asp Leu Asn Val Val Gly Gly Ala Ser Phe Val Ala Gly Glu Ala
   145 150 155 160
   Tyr Asn Thr Asp Gly Asn Gly His Gly Thr His Val Ala Gly Thr Val
   165 170 175
   Ala Ala Leu Asp Asn Thr Thr Gly Val Leu Gly Val Ala Pro Ser Val
   180 185 190
   Ser Leu Tyr Ala Val Lys Val Leu Asn Ser Ser Gly Ser Gly Thr Tyr
   195 200 205
   Ser Gly Ile Val Ser Gly Ile Glu Trp Ala Thr Thr Asn Gly Met Asp
   210 215 220
   Val Ile Asn Met Ser Leu Gly Gly Pro Ser Gly Ser Thr Ala Met Lys
   225 230 235 240
   Gln Ala Val Asp Asn Ala Tyr Ala Arg Gly Val Val Val Val Ala Ala
   245 250 255
   Ala Gly Asn Ser Gly Ser Ser Gly Asn Thr Asn Thr Ile Gly Tyr Pro
   260 265 270
   Ala Lys Tyr Asp Ser Val Ile Ala Val Gly Ala Val Asp Ser Asn Ser
   275 280 285
   Asn Arg Ala Ser Phe Ser Ser Val Gly Ala Glu Leu Glu Val Met Ala
   290 295 300
   Pro Gly Ala Gly Val Tyr Ser Thr Tyr Pro Thr Ser Thr Tyr Ala Thr
   305 310 315 320
   Leu Asn Gly Thr Ser Met Ala Ser Pro His Val Ala Gly Ala Ala Ala
   325 330 335
   Leu Ile Leu Ser Lys His Pro Asn Leu Ser Ala Ser Gln Val Arg Asn
   340 345 350
   Arg Leu Ser Ser Thr Ala Thr Tyr Leu Gly Ser Ser Phe Tyr Tyr Gly
           355                 360                 365
   Lys Gly Leu Ile Asn Val Glu Ala Ala Ala Gln
       370                 375
<210> 3
   <211> 275
   <212> PRT
   <213> Bacillus amyloliquefaciens
<400> 3
<210> 4
   <211> 387
   <212> PRT
   <213> Bacillus licheniformis
<400> 4
   Ser Glu Gln Glu Asn Glx Ala Ser Met Met Arg Lys Lys Ser Phe Trp
   1 5 10 15
   Leu Gly Met Leu Thr Ala Phe Met Leu Val Phe Thr Met Ala Phe Ser
   20 25 30
   Asp Ser Ala Ser Ala Ala Gln Pro Ala Lys Asn Val Glu Lys Asp Tyr
   35 40 45
   Ile Val Gly Phe Lys Ser Gly Val Lys Thr Ala Ser Val Lys Lys Asp
   50 55 60
   Ile Ile Lys Glu Ser Gly Gly Lys Val Asp Lys Gln Phe Arg Ile Ile
   65 70 75 80
   Asn Ala Ala Lys Ala Lys Leu Asp Lys Glu Ala Leu Lys Glu Val Lys
   85 90 95
   Asn Asp Pro Asp Val Ala Tyr Val Glu Glu Asp His Val Ala His Ala
   100 105 110
   Leu Ala Gln Thr Val Pro Tyr Gly Ile Pro Leu Ile Lys Ala Asp Lys
   115 120 125
   Val Gln Ala Gln Gly Phe Lys Gly Ala Asn Val Lys Val Ala Val Leu
       130                 135                 140
   Asp Thr Gly Ile Gln Ala Ser His Pro Asp Leu Asn Val Val Gly Gly
   145                 150                 155                 160
   Ala Ser Phe Val Ala Gly Glu Ala Tyr Asn Thr Asp Gly Asn Gly His
                   165                 170                 175
   Gly Thr His Val Ala Gly Thr Val Ala Ala Leu Asp Asn Thr Thr Gly
               180                 185                 190
   Val Leu Gly Val Ala Pro Ser Val Ser Leu Tyr Ala Val Lys Val Leu
           195                 200                 205
   Asn Ser Ser Gly Ser Gly Ser Tyr Ser Gly Ile Val Ser Gly Ile Glu
       210                 215                 220
   Trp Ala Thr Thr Asn Gly Met Asp Val Ile Asn Met Ser Leu Gly Gly
   225                 230                 235                 240
   Ala Ser Gly Ser Thr Ala Met Lys Gln Ala Val Asp Asn Ala Tyr Ala
                   245                 250                 255
   Arg Gly Val Val Val Val Ala Ala Ala Gly Asn Ser Gly Ser Ser Gly
               260                 265                 270
   Asn Thr Asn Thr Ile Gly Tyr Pro Ala Lys Tyr Asp Ser Val Ile Ala
           275                 280                 285
   Val Gly Ala Val Asp Ser Asn Ser Asn Arg Ala Ser Phe Ser Ser Val
       290                 295                 300
   Gly Ala Glu Leu Glu Val Met Ala Pro Gly Ala Gly Val Tyr Ser Thr
   305                 310                 315                 320
   Tyr Pro Thr Asn Thr Tyr Ala Thr Leu Asn Gly Thr Ser Met Ala Ser
                   325                 330                 335
   Pro His Val Ala Gly Ala Ala Ala Leu Ile Leu Ser Lys His Pro Asn
               340                 345                 350
   Leu Ser Ala Ser Gln Val Arg Asn Arg Leu Ser Ser Thr Ala Thr Tyr
           355                 360                 365
   Leu Gly Ser Ser Phe Tyr Tyr Gly Lys Gly Leu Ile Asn Val Glu Ala
       370                 375                 380
   Ala Ala Gln
   385
<210> 5
   <211> 379
   <212> PRT
   <213> Bacillus licheniformis
<400> 5
   Met Met Arg Lys Lys Ser Phe Trp Leu Gly Met Leu Thr Ala Phe Met
   1               5                   10                  15
   Leu Val Phe Thr Met Ala Phe Ser Asp Ser Ala Ser Ala Ala Gln Pro
               20                  25                  30
   Ala Lys Asn Val Glu Lys Asp Tyr Ile Val Gly Phe Lys Ser Gly Val
           35                  40                  45
   Lys Thr Ala Ser Val Lys Lys Asp Ile Ile Lys Glu Ser Gly Gly Lys
       50                  55                  60
   Val Asp Lys Gln Phe Arg Ile Ile Asn Ala Ala Lys Ala Lys Leu Asp
   65                  70                  75                  80
   Lys Glu Ala Leu Lys Glu Val Lys Asn Asp Pro Asp Val Ala Tyr Val
                   85                  90                  95
   Glu Glu Asp His Val Ala His Ala Leu Ala Gln Thr Val Pro Tyr Gly
               100                 105                 110
   Ile Pro Leu Ile Lys Ala Asp Lys Val Gln Ala Gln Gly Phe Lys Gly
           115                 120                 125
   Ala Asn Val Lys Val Ala Val Leu Asp Thr Gly Ile Gln Ala Ser His
       130                 135                 140
   Pro Asp Leu Asn Val Val Gly Gly Ala Ser Phe Val Ala Gly Glu Ala
   145                 150                 155                 160
   Tyr Asn Thr Asp Gly Asn Gly His Gly Thr His Val Ala Gly Thr Val
                   165                 170                 175
   Ala Ala Leu Asp Asn Thr Thr Gly Val Leu Gly Val Ala Pro Ser Val
               180                 185                 190
   Ser Leu Tyr Ala Val Lys Val Leu Asn Ser Ser Gly Ser Gly Thr Tyr
           195                 200                 205
   Ser Gly Ile Val Ser Gly Ile Glu Trp Ala Thr Thr Asn Gly Met Asp
       210                 215                 220
   Val Ile Asn Met Ser Leu Gly Gly Pro Ser Gly Ser Thr Ala Met Lys
   225                 230                 235                 240
   Gln Ala Val Asp Asn Ala Tyr Ala Arg Gly Val Val Val Val Ala Ala
                   245                 250                 255
   Ala Gly Asn Ser Gly Ser Ser Gly Asn Thr Asn Thr Ile Gly Tyr Pro
               260                 265                 270
   Ala Lys Tyr Asp Ser Val Ile Ala Val Gly Ala Val Asp Ser Asn Ser
           275                 280                 285
   Asn Arg Ala Ser Phe Ser Ser Val Gly Ala Glu Leu Glu Val Met Ala
       290                 295                 300
   Pro Gly Ala Gly Val Tyr Ser Thr Tyr Pro Thr Ser Thr Tyr Ala Thr
   305                 310                 315                 320
   Leu Asn Gly Thr Ser Met Ala Ser Pro His Val Ala Gly Ala Ala Ala
                   325                 330                 335
   Leu Ile Leu Ser Lys His Pro Asn Leu Ser Ala Ser Gln Val Arg Asn
               340                 345                 350
   Arg Leu Ser Ser Thr Ala Thr Tyr Leu Gly Ser Ser Phe Tyr Tyr Gly
           355                 360                 365
   Lys Gly Leu Ile Asn Val Glu Ala Ala Ala Gln
       370                 375
<210> 6
   <211> 269
   <212> PRT
   <213> Bacillus Lentus
<400> 6
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Glu Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 7
   <211> 275
   <212> PRT
   <213> Bacillus amyloliquefaciens
<400> 7
   Ala Gln Ser Val Pro Tyr Gly Val Ser Gln Ile Lys Ala Pro Ala Leu
   1               5                   10                  15
   His Ser Gln Gly Tyr Thr Gly Ser Asn Val Lys Val Ala Val Ile Asp
               20                  25                  30
   Ser Gly Ile Asp Ser Ser His Pro Asp Leu Lys Val Ala Gly Gly Ala
           35                  40                  45
   Ser Met Val Pro Ser Glu Thr Asn Pro Phe Gln Asp Asn Asn Ser His
       50                  55                  60
   Gly Thr His Val Ala Gly Thr Val Ala Ala Leu Asn Asn Ser Ile Gly
   65                  70                  75                  80
   Val Leu Gly Val Ala Pro Ser Ala Ser Leu Tyr Ala Val Lys Val Leu
                   85                  90                  95
   Gly Ala Asp Gly Ser Gly Gln Tyr Ser Trp Ile Ile Asn Gly Ile Glu
               100                 105                 110
   Trp Ala Ile Ala Asn Asn Met Asp Val Ile Asn Met Ser Leu Gly Gly
           115                 120                 125
   Pro Ser Gly Ser Ala Ala Leu Lys Ala Ala Val Asp Lys Ala Val Ala
       130                 135                 140
   Ser Gly Val Val Val Val Ala Ala Ala Gly Asn Glu Gly Thr Ser Gly
   145                 150                 155                 160
   Ser Ser Ser Thr Val Gly Tyr Pro Gly Lys Tyr Pro Ser Val Ile Ala
                   165                 170                 175
   Val Gly Ala Val Asp Ser Ser Asn Gln Arg Ala Ser Phe Ser Ser Val
               180                 185                 190
   Gly Pro Glu Leu Asp Val Met Ala Pro Gly Val Ser Ile Gln Ser Thr
           195                 200                 205
   Leu Pro Gly Asn Lys Tyr Gly Ala Leu Asn Gly Thr Ser Met Ala Ser
       210                 215                 220
   Pro His Val Ala Gly Ala Ala Ala Leu Ile Leu Ser Lys His Pro Asn
   225                 230                 235                 240
   Trp Thr Asn Thr Gln Val Arg Ser Ser Leu Glu Asn Thr Thr Thr Lys
                   245                 250                 255
   Leu Gly Asp Ser Phe Tyr Tyr Gly Lys Gly Leu Ile Asn Val Gln Ala
               260                 265                 270
   Ala Ala Gln
           275